(19) **Europäisches Patentamt / European Patent Office / Office européen des brevets**

(11) **EP 1 937 146 B1**

(12) **FASCICULE DE BREVET EUROPEEN**

(45) Date de publication et mention
de la délivrance du brevet:
**25.04.2012 Bulletin 2012/17**

(21) Numéro de dépôt: **06793714.4**

(22) Date de dépôt: **21.09.2006**

(51) Int Cl.:
*A61B 5/103* (2006.01)          *G01N 27/48* (2006.01)
*G01N 27/30* (2006.01)

(86) Numéro de dépôt international:
**PCT/EP2006/066592**

(87) Numéro de publication internationale:
**WO 2007/036483 (05.04.2007 Gazette 2007/14)**

(54) **Dispositif et procédé de mesure de propriétés physico-chimiques de la peau**

Vorrichtung und Verfahren zur Messung der physikalisch-chemischen Eigenschaften der Haut

Device and method for measuring the physicochemical properties of the skin

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.09.2005 FR 0509758**

(43) Date de publication de la demande:
**02.07.2008 Bulletin 2008/27**

(73) Titulaire: **Pierre Fabre Dermo-Cosmétique
92100 Boulogne-Billancourt (FR)**

(72) Inventeurs:
• **RUFFIEN-CISZAK, Audrey
F-31000 Toulouse (FR)**
• **GROS, Pierre
F-31770 Colomiers (FR)**
• **COMTAT, Maurice
F-81500 Lavaur (FR)**
• **SCHMITT-MILAS, Anne-Marie
F-31770 Tournefeuille (FR)**
• **QUESTEL, Emmanuel
F-31100 Toulouse (FR)**
• **CASAS, Christiane
F-31460 Maureville (FR)**
• **REDOULES, Daniel
F-31300 Toulouse (FR)**

(74) Mandataire: **Ahner, Francis et al
Cabinet Regimbeau
20, rue de Chazelles
75847 Paris Cedex 17 (FR)**

(56) Documents cités:
**WO-A2-2004/030537     FR-A- 2 845 264
US-A- 5 185 922**

• **RUFFIEN-CISZAK ET AL: "Exploration of the global antioxidant capacity of the stratum corneum by cyclic voltammetry" JOURNAL OF PHARMACEUTICAL AND BIOMEDICAL ANALYSIS, NEW YORK, NY, US, vol. 40, no. 1, 30 août 2005 (2005-08-30), pages 162-167, XP005239461 ISSN: 0731-7085**
• **RUFFIEN-CISZAK A ET AL: "Exploration of the global antioxidant capacity of the stratum corneum by cyclic voltammetry", JOURNAL OF PHARMACEUTICAL AND BIOMEDICAL ANALYSIS, NEW YORK, NY, US, vol. 40, no. 1, 23 January 2006 (2006-01-23), pages 162-167, XP025145627, ISSN: 0731-7085, DOI: DOI: 10.1016/J.JPBA.2005.05.035 [retrieved on 2006-01-23]**

EP 1 937 146 B1

**Description**

DOMAINE TECHNIQUE GENERAL

La présente invention concerne un dispositif de mesure des propriétés physico-chimiques de la peau, notamment son pH et son état d'oxydation, comportant un multipotentiostat de génération, de régulation et de traitement de tensions électriques, relié à des moyens de mesure de propriétés de la peau.

Elle concerne également un procédé d'utilisation du dispositif de mesure.

ETAT DE L'ART

La peau fait partie des tissus en perpétuelle interaction avec l'environnement. Sa fonction principale est de protéger le corps humain contre les agressions extérieures d'origines chimique, mécanique, microbienne et lumineuse.

Le vieillissement cutané est en grande partie lié, comme beaucoup d'autres processus dégénératifs, à l'accumulation d'agressions oxydantes.

La peau a développé des systèmes de défense enzymatiques, comme par exemple la superoxyde dismutase (SOD), la glutathion peroxydase et la catalase, et non enzymatiques, comme par exemple les vitamines E et C, l'acide urique et le glutathion.

Il est connu qu'une exposition au stress provoque une diminution des capacités antioxydantes de la peau.

Généralement, le stress oxydant est considéré comme étant la résultante d'une rupture de l'équilibre antioxydants/prooxydants. Ce déséquilibre peut avoir plusieurs origines comme une carence en antioxydants ou en protéines porteuses directement d'une activité antioxydante. Par exemple, une irradiation de la couche cornée à des doses subérythémales entraîne une diminution d'$\alpha$-tocophérol. Des carences en vitamine C ont également été rapportées suite à des expositions répétées à l'ozone.

Cependant, une des questions majeures reste celle du lien entre le stress oxydant et les capacités réductrices de la surface cutanée.

De même, il est intéressant de connaître l'évolution des pH locaux sous l'influence de ou en réponse à ce stress. Des modifications de pH peuvent en effet traduire une possible régulation des enzymes antioxydantes de la fonction barrière.

Il n'existe pas de dispositif électrochimique permettant des mesures directes, localisées et simultanées du pH et de marqueurs des capacités antioxydantes de la peau afin d'étudier l'influence de l'environnement sur le potentiel antioxydant cutané.

Le document FR-A- 2 845 264 décrit un dispositif de mesure de propriétés physico-chimiques de la peau, notamment son pH et son état d'oxydation, comportant des moyens de génération, de régulation et de traitement de tensions électriques reliés à des moyens de mesure de propriétés de la peau comportant une micro-électrode de travail, une électrode de référence et une électrode auxiliaire. L'électrode de référence est une électrode au chlorure d'argent Ag/AgCl/Cl⁻.

L'article "Exploration of the global antioxidant capacity of the stratum corneum by cyclic voltammetry", de Ruffien-Ciszak et Al, JOURNAL OF PHARMACEUTICAL AND BIOMEDICAL ANALYSIS, août 2005, divulgue un dispositif de mesure de propriétés physico-chimiques de la peau utilisant une ou plusieurs microélectrodes de travail, une électrode auxiliaire et une électrode de référence. L'électrode de référence consiste en une électrode au calomel saturé connectée à une sorte de "stylo feutre" utilisé comme "Luggin capillary" pour faciliter l'application sur la peau.

PRESENTATION DE L'INTENTION

L'invention propose de pallier au moins un des inconvénients de l'art antérieur.

A cet effet, on propose selon l'invention un dispositif de mesure des propriétés physico-chimiques de la peau, selon la revendication 1.

L'invention est avantageusement complétée par les caractéristiques suivantes, prises seules ou en une quelconque de leur combinaison techniquement possible :

- les moyens de mesure comportent quatre microélectrodes de travail ;
- le fil métallique des microélectrodes de travail est en platine, en or, en argent et en tungstène ou palladium respectivement
- les quatre électrodes de travail sont disposées selon un cercle, le centre du cercle étant occupé par la microélectrode de référence ;
- le cercle a un rayon de l'ordre de 1 cm, l'aire de mesure étant inférieure à $10^{-2}$ mm² ; et
- le diamètre extérieur moyen de l'extrémité libre de chaque microélectrode de travail est sensiblement égal à 120 $\mu$m, le diamètre intérieur moyen étant sensiblement égal à 50 $\mu$m.

L'invention concerne également un procédé d'utilisation du dispositif de mesure selon la revendication 7. Les revendications dépendantes 8-13 définissent des modes de réalisation préférés du procédé selon l'invention.

L'invention présente de nombreux avantages.

On propose un dispositif analytique composé d'une série de microélectrodes électrochimiques pour l'analyse des propriétés acido-basiques et d'oxydo-réduction de la peau. Les mesures permettent simultanément de déterminer le pH et de détecter la présence d'espèces ioniques et de molécules antioxydantes, parmi lesquelles l'ion chlorure, l'acide ascorbique, l'acide urique et le glutathion.

Les mesures s'effectuent à l'aide de microélectrodes de matériaux différents qui permettent des détections d'espèces et de molécules différentes simultanément. L'invention permet non seulement d'informer sur l'état redox global de la peau, mais également de détecter la présence de plusieurs antioxydants cutanés.

L'invention utilise, pour les références de mesure, une seule et même microélectrode dite pseudo-électrode de référence. Ainsi, chaque circuit d'électrolyse n'est composé que de deux microélectrodes. La technologie utilisée est peu onéreuse.

Les mesures sont réalisées directement à la surface de la peau, sans addition de solvant ou de gel et sans modification de surface des électrodes. Le procédé analytique est non invasif. Les microélectrodes sont simplement déposées à la surface de la peau et aucune biopsie n'est nécessaire.

L'expérience est menée sur une surface de peau inférieure à $10^{-2}$ mm$^2$. Toutes les électrodes sont miniaturisées ; les fils métalliques ont un diamètre de 50 μm. Un dispositif selon l'invention permet de s'affranchir d'une macroélectrode de référence disponible dans le commerce.

Le temps d'analyse est d'une à deux minutes. Les résultats sont obtenus en direct. Il n'existe quasiment pas de délai entre la mesure et le résultat. Ceci permet d'envisager de suivre la dynamique des processus métaboliques de la peau et d'enregistrer en temps réel la réponse du milieu cutané à diverses agressions.

L'invention assure ainsi une très bonne résolution spatio-temporelle.

PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :

- la figure 1 est une représentation schématique d'un montage possible d'un dispositif de mesure selon l'invention ;
- la figure 2 est une représentation schématique d'une coupe longitudinale d'un mode de réalisation possible de microélectrode ;
- la figure 3 est une représentation schématique d'une coupe radiale d'un mode de réalisation de la figure 2 ;
- la figure 4 est une représentation schématique de l'évolution au cours du temps du potentiel à courant nul d'une microélectrode de tungstène ou palladium appliquée sur un avant-bras d'un patient ;
- les figures 5a, 5b et 5c sont des représentations schématiques de voltammogrammes cycliques obtenus avec les microélectrodes appliquées sur l'avant-bras d'un patient pour respectivement une microélectrode de platine, une microélectrode d'or et une microélectrode d'argent ;
- la figure 6 est une représentation schématique de l'évolution du potentiel à courant nul d'une microélectrode de tungstène ou palladium en fonction du pH à la surface de la peau mesurée grâce à une électrode à membrane de verre ;
- les figures 7a et 7b sont des représentations schématiques de voltammogrammes obtenus avec une microélectrode de platine déposée sur la peau saine (traits pointillés) et sur la peau traitée (traits pleins) respectivement avec une solution d'acide ascorbique 0,1 mol L$^{-1}$ et avec une solution d'acide urique 0,1 mol L$^{-1}$ ; et
- la figure 8 est une représentation schématique de voltammogrammes obtenus avec une microélectrode d'or déposée sur la peau saine (traits pointillés) et sur la peau traitée (trait plein) avec une solution de glutathion 0,1 mol L$^{-1}$.

DESCRIPTION DETAILLEE

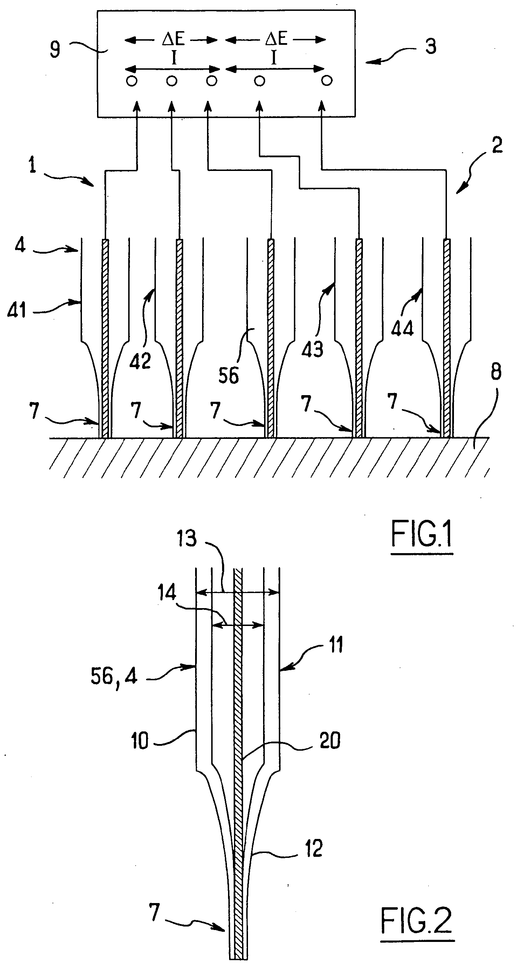

La figure 1 est une représentation schématique d'un montage possible d'un dispositif 1 de mesure.

Le dispositif 1 comporte principalement deux parties : une partie 2 est apte à effectuer les mesures sur une peau 8 d'un patient ; une partie 3 comprend un multipotentiostat 9 apte à générer et à réguler des tensions électriques vers la partie 2. Le dispositif 1 comporte de plus des moyens d'acquisition et de traitement des résultats des mesures reçues de la partie 2.

La partie 2 de mesure comporte deux éléments principaux : au moins une microélectrode de travail 4 et une microélectrode 56 utilisée comme pseudo-électrode de référence. La partie 2 comporte préférentiellement une pluralité de microélectrodes 4 de travail.

La pluralité comporte préférentiellement une microélectrode 41 d'argent, et/ou une microélectrode 42 d'or, et/ou une microélectrode 43 de platine, et/ou une microélectrode 44 de tungstène ou de palladium, utilisées comme électrode 4 de travail. Chaque microélectrode 4 de travail comporte une extrémité libre 7 apte à être directement posée sur la peau d'un patient.

La microélectrode 56 se présente sous la forme d'une microélectrode d'argent par rapport à laquelle tous les potentiels sont exprimés.

Chaque microélectrode 4 de travail et la microélectrode 56 de référence sont reliées à la partie 3. La partie 3 comporte un multipotentiostat 9.

Le multipotentiostat 9 permet d'appliquer une différence de potentiel variable ΔE entre chacune des microélectrodes 4 et la microélectrode 56. Le multipotentiostat 9 permet également de mesurer le courant 1 traversant chaque circuit formé par les microélectrodes 4 et la microélectrode 56.

Lorsque l'on veut effectuer une mesure de pH et d'état d'oxydation, l'extrémité 7 de chaque microélectrode 4 est positionnée à la surface de la peau 8. Le positionnement des microélectrodes 4 est effectué préférentiellement à l'aide d'un micromanipulateur de précision non représenté sur la figure 1. Les microélectrodes 4 sont disposées selon un cercle de rayon 1 cm. La microélectrode 56 de référence est placée au centre du cercle formé par les microélectrodes 41 et/ou 42 d'or et/ou 43 et/ou 44.

Lors d'une mesure, deux types d'expériences sont réalisés simultanément.

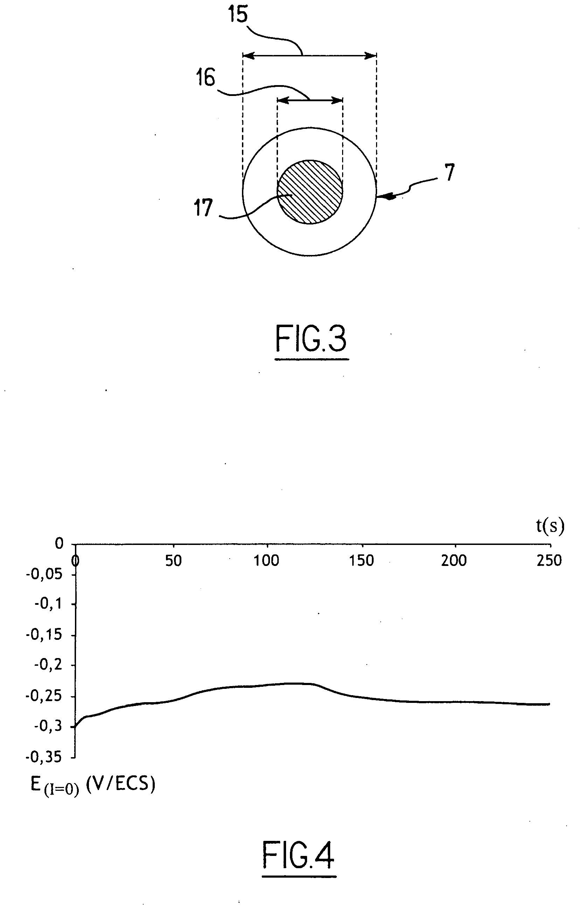

Le premier type de mesure réside dans la mesure de la différence de potentiel $\Delta E$ à courant I nul entre une microélectrode de travail 4 et la microélectrode 56. Préférentiellement, la microélectrode 4 est la microélectrode 44 de tungstène ou de palladium. Cette mesure est effectuée durant 150 secondes à 300 secondes. Un exemple de courbe obtenue par cette mesure est visible à la figure 4.

La détermination du pH découle de la mesure du potentiel à courant nul mesuré entre la microélectrode 44 et la microélectrode 56.

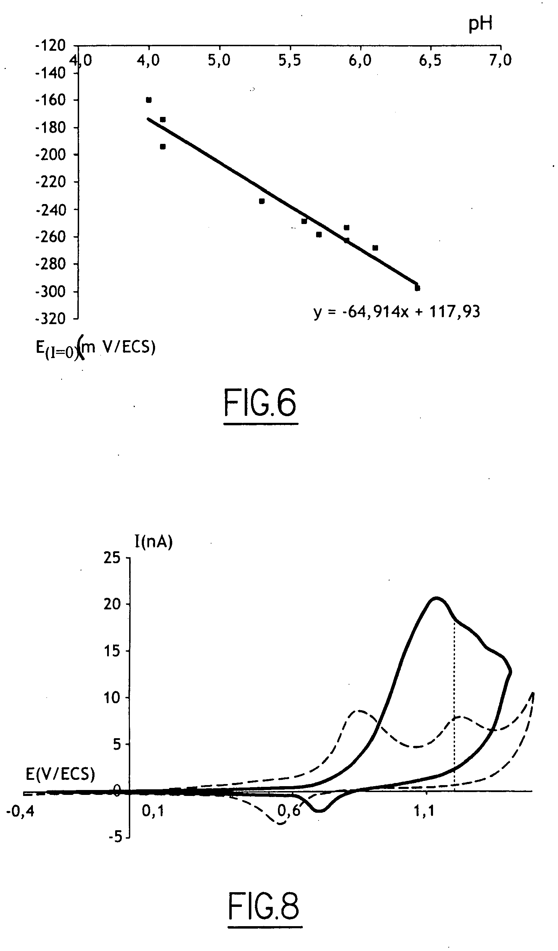

Sur la figure 6 est présentée l'évolution du potentiel à courant nul de la microélectrode 44 en fonction du pH à la surface de la peau. Le potentiel à courant nul de la microélectrode diminue lorsque le pH augmente. La relation donnant E en fonction du pH est par exemple :

$$pH = -64.914\ E + 117.93$$

Le graphe de la figure 6 est établi lors d'un calibrage préalable du dispositif de mesure. On construit cette courbe en corrélant la différence de potentiel $\Delta E$ à courant nul mesurée sur la peau de différents sujets, au pH de la peau mesuré par une électrode à membrane de verre connue en soi par l'homme du métier. Cette courbe est construite avec un grand nombre de pH en abscisse, auxquels correspondent des potentiels mesurés. Elle est ensuite mémorisée par des moyens compris dans le dispositif d'acquisition et de traitement des résultats des mesures.

Ainsi, lors d'une utilisation postérieure de l'appareil, c'est-à-dire lors d'une mesure, l'appareil effectue la mesure de la différence de potentiel $\Delta E$ à courant nul sur la peau afin d'obtenir la courbe selon la figure 4 (sur la figure 6, cette valeur est à peu près égale à -0,26V). A partir de la courbe de correspondance selon la figure 6, l'appareil donne à l'utilisateur la valeur du pH de la peau.

Le deuxième type de mesure consiste à enregistrer un voltammogramme cyclique d'autres microélectrodes 4 pour des potentiels compris entre -0,8V et 1,5V, à une vitesse de balayage comprise entre 10mV/s et 1V/s. Préférentiellement, la vitesse de balayage est de 50 mV/s.

Un voltammogramme est une courbe qui indique le courant I d'électrolyse traversant le circuit constitué d'une microélectrode de travail 4 et de la microélectrode 56, en fonction de la différence de potentiel $\Delta E$ appliquée.

La détection des différentes espèces est effectuée par l'intermédiaire du potentiel de demi-vague ou du potentiel de pic du signal ampérométrique d'oxydation sur les voltammogrammes. Ce potentiel est caractéristique de chaque système oxydo-réducteur.

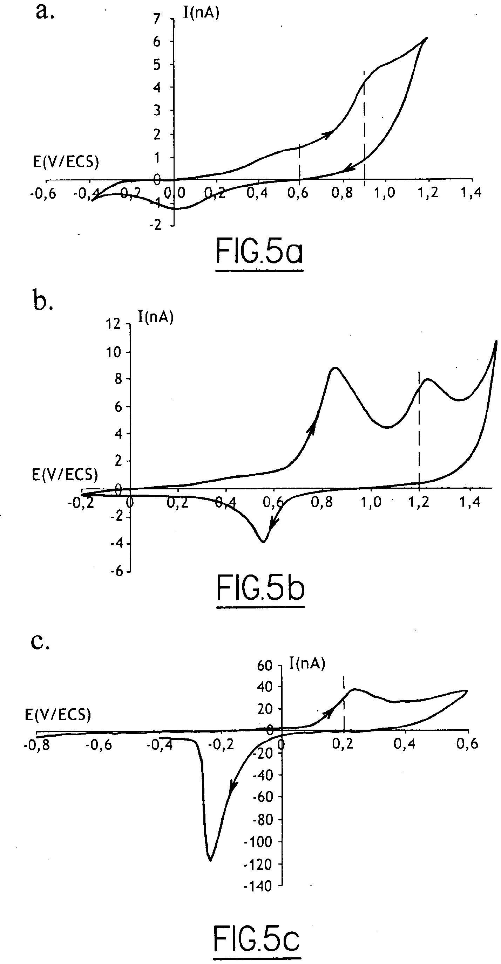

Préférentiellement, pour la microélectrode 43 de platine on effectue un cycle de potentiel entre -0,4 $V/E_{ref}$ et 1,2 $V/E_{ref}$. Un exemple d'une courbe obtenue est montré sur la figure 5a.

L'acide ascorbique est détecté par la mesure du courant d'oxydation enregistré à la microélectrode 43 à un potentiel de demi-vague sensiblement autour de 0,4 $V/E_{ref}$. Préférentiellement, la mesure quantitative est effectuée à 0,6 $V/E_{ref}$.

L'apparition d'une intensité d'oxydation débutant à environ 0,7$V/E_{ref}$ avec un plateau à 0,9$V/E_{ref}$ sur la microélectrode 43 de platine prouve la présence d'acide urique sur la peau. L'acide urique peut donc être détecté à un potentiel de demi-vague sensiblement autour de 0,8 $V/E_{ref}$. Préférentiellement, la quantification de l'acide urique est effectuée par la mesure du courant d'oxydation enregistré à la microélectrode 43 de platine au potentiel de 0,9 $V/E_{ref}$, après soustraction du courant d'oxydation au potentiel de 0,6 $V/E_{ref}$ correspondant à l'acide ascorbique.

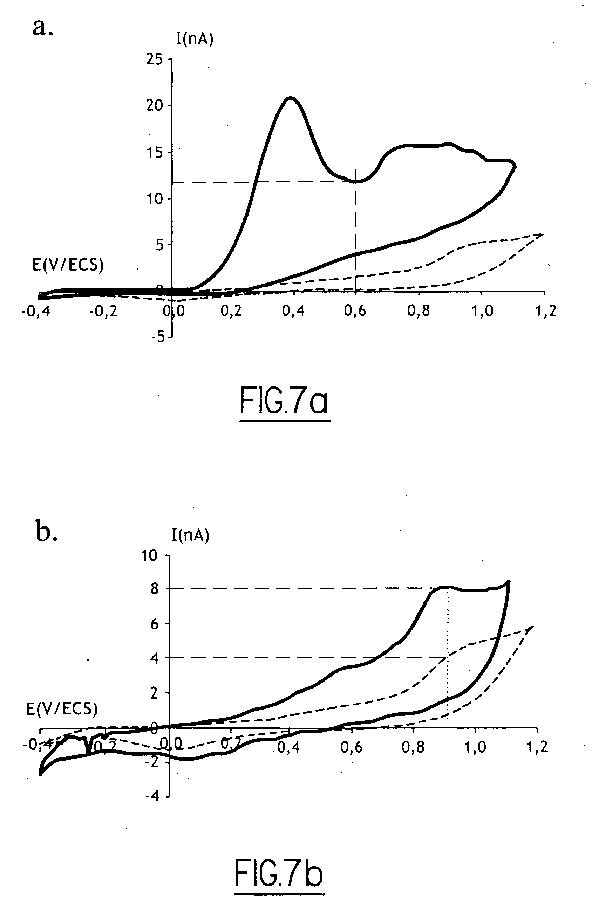

Les figures 7a et 7b sont des représentations schématiques de voltammogrammes obtenus avec une microélectrode 43 de platine déposée sur la peau saine (traits pointillés) et sur la peau traitée (traits pleins) respectivement avec une solution d'acide ascorbique 0,1 mol $L^{-1}$ (figure 7a) et avec une solution d'acide urique 0,1 mol $L^{-1}$ (figure 7b).

L'intensité du courant à 0,6$V/E_{ref}$ sur la microélectrode 43 de platine est reliée à la quantité d'acide ascorbique présent : plus celle-ci est importante, plus l'intensité d'oxydation est forte.

De même, l'intensité du courant à 0,9$V/E_{ref}$ sur électrode de platine, après soustraction du courant à 0,6$V/E_{ref}$, est reliée à la quantité d'acide urique présent sur la peau : plus celle-ci est importante, plus l'intensité d'oxydation est forte.

Préférentiellement, pour la microélectrode 42 d'or on effectue un cycle de potentiel entre -0,2 $V/E_{ref}$ et 1,5 $V/E_{ref}$. Un exemple d'une courbe obtenue est montré sur la figure 5b. Le glutathion est quantifié par la mesure du courant d'oxydation enregistré à la microélectrode 42 au potentiel de 1,2 $V/E_{ref}$.

La figure 8 est une représentation schématique de voltammogrammes obtenus avec une microélectrode 42 d'or déposée sur la peau saine (traits pointillés) et sur la peau traitée (traits pleins) avec une solution de glutathion 0,1 mol $L^{-1}$.

L'intensité du courant d'oxydation à 1,2$V/E_{ref}$ sur la microélectrode 42 d'or est reliée à la quantité de glutathion présent : plus celle-ci est importante, plus l'intensité d'oxydation est forte.

Préférentiellement, pour la microélectrode 41 d'argent on effectue un cycle de potentiel entre -0,8 $V/E_{ref}$ et 0,6 $V/E_{ref}$. Un exemple d'une courbe obtenue est montré sur la figure 5c. Les ions chlorures sont quantifiés par la mesure du courant d'oxydation enregistré avec la microélectrode 41 au potentiel de demi-vague de 0,2 V/E$_{ref}$.

A ce potentiel, l'argent seul ne peut s'oxyder. La présence d'un courant d'oxydation à un potentiel si bas nécessite obligatoirement la présence des ions chlorures.

L'intensité du courant à 0,2V/E$_{ref}$ sur la microélectrode 41 d'argent est reliée à la quantité d'ions chlorures présents : plus celle-ci est importante, plus l'intensité d'oxydation est forte.

La construction des voltammogrammes des courbes des figures 5a-c permet de détecter la présence et de quantifier la concentration d'acide ascorbique, d'acide urique, de glutathion et d'ions chlorures.

En absence d'une ou de l'autre des espèces, l'intensité d'oxydation correspondante est nulle.

Sur les figures 5a à 5c, on note la présence de flèches qui indiquent le sens d'obtention de la courbe lors du balayage à partir du potentiel -0,8V et 1,5V (partie haute de la courbe, trajet correspondant à l'oxydation) puis de 1,5V vers -0,8V (partie basse de la courbe, trajet correspondant à la réduction).

Le procédé analytique permet d'effectuer directement des mesures à la surface de la peau, sans addition de solvant ni de gel, et sans modification de surface du capteur.

On va maintenant décrire plus en détails les électrodes de la partie 2 du dispositif 1.

La figure 2 est une représentation schématique d'une coupe longitudinale d'un mode de réalisation possible de microélectrode de travail 4 ou de référence 56.

Sur la figure 2, la microélectrode comporte un capillaire 10 isolant, préférentiellement en verre, dans lequel s'étend un fil 20 de platine, d'argent, d'or, de tungstène ou de palladium en fonction de la microélectrode que l'on veut obtenir.

Le capillaire 10 comporte une première partie 11 reliée à la partie 3 du dispositif, une autre partie 12 s'étendant à partir de la partie 11 vers l'extrémité libre 7 de la microélectrode 4 ou 56.

La partie 11 forme un tube à symétrie de révolution circulaire. Le diamètre externe 13 de la partie 11 est sensiblement égal à 1,2 mm par exemple. Le diamètre interne 14 est sensiblement égal à 0,7 mm par exemple.

La partie 12 est également à symétrie de révolution, mais de section droite de plus en plus faible, à partir de la partie 11 vers l'extrémité libre 7. La figure 3 montre que le diamètre externe 15 de l'extrémité 7 est de l'ordre de 120 μm environ. Le diamètre externe 16 du fil de platine 20 à l'extrémité 7 est de l'ordre de 50 μm.

Les microélectrodes permettent de déterminer simultanément le pH et l'état d'oxydation sur une surface de peau inférieure à 10$^{-2}$ mm$^2$, pour un temps d'analyse inférieur à cinq minutes, par exemple compris entre une et deux minutes et préférentiellement inférieur à une minute. Il assure ainsi une très bonne résolution analytique dans l'espace et dans le temps.

La composition du fil 20 est préférentiellement :

- Pt 90% - Ir 10% pour la microélectrode 43 de platine pour garantir une bonne résistance mécanique ;
- Au 99.99% pour la microélectrode 42 d'or ;
- W ou Pd 99.95% pour la microélectrode 44 de tungstène ou de palladium ;
- Ar 99.99% pour la microélectrode 41 ou 56 d'argent.

Lors de la fabrication de la microélectrode, le fil 20 est introduit dans le tube 10 à section cylindrique de révolution. Le fil 20 est ensuite étiré à l'aide d'une microétireuse. L'extrémité 7 de la microélectrode 4 est alors chauffée de façon à parfaitement enrober le fil de platine de verre, ce qui donne la forme de la partie 12 de la microélectrode 4.

L'extrémité 7 est ensuite poncée sur une résine diamantée de façon à obtenir un disque 17 de platine plan, le disque étant visible à la figure 3.

La surface des disques de platine, d'or et d'argent est traitée avant chaque mesure en faisant subir aux microélectrodes des cycles répétés de potentiel à la vitesse de balayage de 50 mV/s comme suit.

Pour la microélectrode 43 de platine : cycles de potentiel entre -0,6 V/E$_{ref}$ et 1,0 V/E$_{ref}$ dans une solution tamponnée de phosphate 0,1 mol L$^{-1}$ à pH=7,0.

Pour la microélectrode 42 d'or: cycles de potentiel entre -0,5 V/E$_{ref}$ et 1,4 V/E$_{ref}$ dans une solution tamponnée de phosphate 0,1 mol L$^{-1}$ à pH=7,0.

Pour la microélectrode 41 d'argent : cycles de potentiel entre -1,0 V/E$_{ref}$ et 0,4 V/E$_{ref}$ dans une solution de nitrate de potassium KNO$_3$ / HNO$_3$ 0,1 mol L$^{-1}$ à pH=3,5.

La surface du disque de tungstène ou de palladium est préparée en portant la microélectrode 44 au potentiel de 1,0 V/E$_{ref}$ dans une solution tamponnée de phosphate 0,1 mol L$^{-1}$ à pH=7,0 pendant 2 minutes. Il se forme des oxydes de tungstène, en particulier WO$_3$, ou des oxydes de palladium.

La microélectrode 56 de référence est préparée en portant la microélectrode d'argent à une intensité de 30 nA dans une solution de chlorure de potassium NaCl 3 mmol.L$^{-1}$, à pH=4, pendant 5 minutes. Il se forme un précipité de chlorure d'argent AgCl.

**Revendications**

1. Dispositif (1) de mesure des propriétés physico-chimiques de la peau (8), notamment son pH et son état d'oxydation, comportant un multipotentiostat (9) de génération, de régulation et de traitement de tensions électriques, relié à des moyens (2) de mesure de propriétés de la peau, comportant au moins deux microélectrodes (4) de travail et une microélectrode (56) de référence, chaque microélectrode (4) de travail comportant un tube capillaire (10) isolant électrique à l'intérieur duquel s'étend un fil métallique (20) dont l'extrémité libre (7) forme un disque (17) apte à être mis en contact direct avec la peau lors des mesures, la microélectrode (56) de référence comportant un tube capillaire(10) isolant électrique à l'interieur duquel s'étend un fil d'argent(20) dont l'extrémité libre (7) forme un disque (17) apte à être mis en contact avec la peau lors des mesures, la microélectrode (56) de référence comportant en outre un précipité de chlorure d'argent AgCl sur l'extrémité libre (7)

**2.** Dispositif selon la revendication 1, dans lequel les moyens (2) de mesure comportent quatre microélectrodes (41, 42, 43, 44) de travail.

**3.** Dispositif selon la revendication 2, dans lequel le fil métallique (20) des microélectrodes (41, 42, 43, 44) de travail est en platine, en or, en argent et en tungstène ou palladium respectivement

**4.** Dispositif selon l'une des revendications 2 ou 3, dans lequel les quatre électrodes (41, 42, 43, 44) de travail forment un cercle, le centre du cercle étant occupé par la microélectrode de référence (56).

**5.** Dispositif selon la revendication 4 , dans lequel le cerde a un 5- Dispositif selon la revendication 4, dans lequel le cercle a un rayon de l'ordre de 1 cm, l'aire de mesure étant inférieure à $10^{-2}$ mm$^2$.

**6.** Dispositif selon l'une des revendications 1 à 5, dans lequel le diamètre extérieur moyen (15) de l'extrémité libre (7) de chaque microélectrode de travail (4) est sensiblement égal à 120 $\mu$m, le diamètre intérieur moyen (16) étant sensiblement égal à 50 $\mu$m.

**7.** Procédé de mesure des propriétés physico-chimiques de la peau, notamment son pH et son état d'oxydation, à l'aide d'un dispositif de mesure (1) selon l'une des revendications 1 à 6 ledit procédé comportant une étape de détermination des valeurs de pH et d'état d'oxydation en fonction de résultats obtenus par des étapes simultanées selon lesquelles :

- on mesure la différence de potentiel ($\Delta$E) à courant (I) nul entre une microélectrode de travail (4) et la microélectrode de référence (56) ;
- on trace simultanément au moins un voltammogramme cyclique entre les potentiels compris entre -0,8V et 1,5V pour les autres microélectrodes, afin d'obtenir la courbe du courant (I) d'électrolyse traversant le circuit constitué de la microélectrode de travail (4) respective et la microélectrode de référence (56), en fonction de la différence de potentiel ($\Delta$E) appliquée entre la microélectrode de travail (4) respective et la microélectrode de référence (56).

**8.** Procédé selon la revendication 7, dans lequel on relie quatre microélectrodes (41, 42, 43, 44) de travail au multipotentiostat (9) et on trace simultanément un voltammogramme cyclique pour trois microélectrodes.

**9.** Procédé selon l'une des revendications 7 ou 8, dans lequel on détermine la valeur du pH à partir de la différence de potentiel à courant nul mesurée entre une microélectrode de travail (44) de tungstène ou palladium et la microélectrode de référence (56), composée d'argent.

**10.** Procédé selon l'une des revendications 7 à 9, dans lequel on détecte la présence :

- d'acide ascorbique et d'acide urique par la mesure d'un courant d'oxydation enregistré à une microélectrode de platine (43) ;
- de glutathion par la mesure d'un courant d'oxydation enregistré à une microélectrode d'or (42); et
- des ions chlorures par la mesure d'un courant d'oxydation enregistré à une microélectrode d'argent (41).

**11.** Procédé selon la revendication 10 dans lequel on quantifie la présence :

- d'acide ascorbique par la mesure du courant d'oxydation enregistré à une microélectrode de platine (43) à un potentiel de 0,6 V/$E_{ref}$. sur un voltammogramme tracé entre -0,4 V/$E_{ref}$ et 1,2 V/$E_{ref}$. ;
- d'acide urique par la mesure du courant d'oxydation enregistré à la microélectrode (43) de platine au potentiel de 0,9 V/$E_{ref}$, après soustraction du courant d'oxydation au potentiel de 0,6 V/$E_{ref}$ correspondant à l'acide ascorbique ;
- de glutathion par la mesure du courant d'oxydation enregistré à une microélectrode d'or (42) au potentiel de 1,2 V/$E_{ref}$. sur un voltammogramme tracé entre -0,2 V/$E_{ref}$ et 1,5 V/$E_{ref}$ ; et
- des ions chlorures par la mesure du courant d'oxydation enregistré à une microélectrode d'argent (41) au potentiel de 0,2 V/$E_{ref}$ sur un voltammogramme tracé entre -0,8 V/$E_{ref}$ et 0,6 V/$E_{ref}$.

**12.** Procédé selon la revendication 11, dans lequel, entre chaque mesure, on prépare les extrémités libres (7) des microélectrodes (41, 42, 43, 44, 56) de la façon suivante :

- pour la microélectrode (43) de platine, on effectue des cycles de potentiel à la vitesse de balayage de 50 mV/s entre -0,6 $V/E_{ref}$ et 1,0 $V/E_{ref}$ dans une solution tamponnée de phosphate 0,1 mol $L^{-1}$ à pH=7,0 ;

- pour la microélectrode (42) d'or, on effectue des cycles de potentiel à la vitesse de balayage de 50 mV/s entre -0,5 $V/E_{ref}$ et 1,4 $V/E_{ref}$ dans une solution tamponnée de phosphate 0,1 mol $L^{-1}$ à pH=7,0.

- pour la microélectrode (41) d'argent, on effectue des cycles de potentiel à la vitesse de balayage de 50 mV/s entre -1,0 $V/E_{ref}$ et 0,4 $V/E_{ref}$ dans une solution de nitrate de potassium $KNO_3$ / $HNO_3$ 0,1 mol $L^{-1}$ à pH=3,5 ;

- pour la microélectrode (44) de tungstène ou palladium, on porte au potentiel de 1,0 $V/E_{ref}$ dans une solution tamponnée de phosphate 0,1 mol $L^{-1}$ à pH=7,0 pendant deux minutes;

- pour la microélectrode (56) de référence, on porte à une intensité de 30 nA dans une solution de chlorure de potassium $NaCl$ 3 $mmol.L^{-1}$, à pH=4, pendant cinq minutes pour former un précipité de chlorure d'argent $AgCl$.

13. Procédé selon l'une des revendications 7 à 12, dans lequel le temps de mesure est inférieur à cinq minutes, préférentiellement inférieur à une minute.

**Claims**

1. Device (1) for measuring the physicochemical properties of skin (8), particularly the pH and oxidation state thereof, comprising a multi-potentiostat (9) for generating, regulating and processing electrical voltages, connected to means (2) for measuring properties of the skin comprising at least two working microelectrodes (4) and one reference microelectrode (56),

    - each working microelectrode (4) comprising an electrically insulating capillary tube (10) inside which a metal wire (20) extends, the free end (7) whereof forms a disk (17) suitable for being placed in direct contact with the skin during the measurements,
    - the reference microelectrode (56) comprising an electrically insulating capillary tube (10) inside which a silver wire (20) extends, the free end (7) whereof forms a disk (17) suitable for being placed in direct contact with the skin during the measurements, the reference microelectrode (56) further comprising a silver chloride $AgCl$ precipitate on the free end (7).

2. Device according to claim 1, wherein the measurement means (2) comprise four working microelectrodes (41, 42, 43, 44).

3. Device according to claim 2, wherein the metal wire (20) of the working microelectrodes (41, 42, 43, 44) is made of platinum, gold, silver and tungsten or palladium, respectively.

4. Device according to any of claims 2 or 3, wherein the four working electrodes (41, 42, 43, 44) form a circle, the centre of the circle being occupied by the reference microelectrode (56).

5. Device according to claim 4, wherein the circle has a radius in the region of 1 cm, the measurement area being less than $10^{-2}$ $mm^2$.

6. Device according to any of claims 1 to 5, wherein the mean outer diameter (15) of the free end (7) of each working microelectrode (4) is substantially equal to 120 $\mu m$, the mean inner diameter (16) being substantially equal to 50 $\mu m$.

7. Method for measuring the physicochemical properties of skin, particularly the pH and oxidation state thereof, using a measurement device (1) according to any of claims 1 to 6, said method comprising a step for determining the pH and oxidation state values on the basis of results obtained by means of simultaneous steps whereby:

    - the potential difference ($\Delta E$) at zero current (I) between a working microelectrode (4) and the reference microelectrode (56) is measured;
    - simultaneously, at least one cyclic voltammogram is plotted between the potentials between -0.8V and 1.5V for the other microelectrodes, so as to obtain the curve of the electrolysis current (I) passing through the circuit consisting of the respective working microelectrode (4) and the reference microelectrode (56), on the basis of the potential difference ($\Delta E$) applied between the respective microelectrode (4) and the reference microelectrode (56).

8. Method according to claim 7, wherein four working microelectrodes (41, 42, 43, 44) are connected to the multi-potentiostat (9) and the cyclic voltammogram is simultaneously plotted for three microelectrodes.

9. Method according to any of claims 7 or 8, wherein the pH value is determined on the basis of the potential difference at zero current measured between a tungsten or palladium working microelectrode (44) and the reference microelectrode (56), made of silver.

10. Method according to any of claims 7 to 9, wherein the presence of the following is detected:

- ascorbic acid and uric acid by measuring an oxidation current recorded on a platinum microelectrode (43);
- glutathione by measuring an oxidation current recorded on a gold microelectrode (42);
- chloride ions by measuring an oxidation current recorded on a silver microelectrode (41).

11. Method according to claim 10, wherein the presence of the following is quantified:

- ascorbic acid by measuring an oxidation current recorded on a platinum microelectrode (43) at a potential of 0.6 $V/E_{ref}$ on a voltammogram plotted between -0.4 $V/E_{ref}$ and 1.2 $V/E_{ref}$;
- uric acid by measuring an oxidation current recorded on a platinum microelectrode (43) at a potential of 0.9 $V/E_{ref}$ after subtracting the oxidation current at a potential of 0.6 $V/E_{ref}$ corresponding to ascorbic acid;
- glutathione by measuring an oxidation current recorded on a gold microelectrode (42) at a potential of 1.2 $V/E_{ref}$ on a voltammogram plotted between -0.2 $V/E_{ref}$ and 1.5 $V/E_{ref}$; and
- chloride ions by measuring an oxidation current recorded on a silver microelectrode (41) at a potential of 0.2 $V/E_{ref}$ on a voltammogram plotted between -0.8 $V/E_{ref}$ and 0.6 $V/E_{ref}$.

12. Method according to claim 11, wherein, between each measurement, the free ends (7) of the microelectrodes (41, 42, 43, 44, 56) are prepared as follows:

- for the platinum microelectrode (43), potential cycles are run at a sweep rate of 50 mV/s between -0.6 $V/E_{ref}$ and 1.0 $V/E_{ref}$ in a 0.1 mol $L^{-1}$ phosphate buffer solution at pH=7.0;
- for the gold microelectrode (42), potential cycles are run at a sweep rate of 50 mV/s between -0.5 $V/E_{ref}$ and 1.4 $V/E_{ref}$ in a 0.1 mol $L^{-1}$ phosphate buffer solution at pH=7.0.
- for the silver microelectrode (41), potential cycles are run at a sweep rate of 50 mV/s between -1.0 $V/E_{ref}$ and 0.4 $V/E_{ref}$ in a 0.1 mol $L^{-1}$ $KNO_3/HNO_3$ potassium nitrate solution at pH=3.5;
- for the tungsten or palladium microelectrode (44), a potential of 1.0 $V/E_{ref}$ is applied in a 0.1 mol $L^{-1}$ phosphate buffer solution at pH=7.0 for two minutes;
- for the reference microelectrode (56), a current of 30 nA is applied in a 3 mmol.$L^{-1}$ NaCl potassium chloride solution at pH=4, for five minutes to form a silver chloride AgCl precipitate.

13. Method according to any of claims 7 to 12, wherein the measurement time is less than five minutes, preferably less than one minute.

**Patentansprüche**

1. Vorrichtung (1) zum Messen der physikalisch-chemischen Eigenschaften der Haut (8), insbesondere ihres pH-Wertes und ihres Oxidationszustands, umfassend einen Multipotentiostat (9) zur Erzeugung, Regelung und Aufbereitung von elektrischen Spannungen, der mit Mitteln (2) zum Messen der Eigenschaften der Haut verbunden ist, welche mindestens zwei Arbeits-Mikroelektroden (4) und eine Referenz-Mikroelektrode (56) umfassen,

- wobei jede Arbeits-Mikroelektrode (4) ein elektrisch isolierendes Kapillarrohr (10) umfasst, in dessen Innern sich ein Metalldraht (20) erstreckt, dessen freies Ende (7) eine Scheibe (17) bildet, die dazu geeignet ist, während der Messungen in direkten Kontakt mit der Haut gebracht zu werden,
- wobei die Referenz-Mikroelektrode (56) ein elektrisch isolierendes Kapillarrohr (10) umfasst, in dessen Innern sich ein Silberdraht (20) erstreckt, dessen freies Ende (7) eine Scheibe (17) bildet, die dazu geeignet ist, während der Messungen in Kontakt mit der Haut gebracht zu werden, wobei die Referenz-Mikroelektrode (56) an dem freien Ende (7) ferner einen Niederschlag von Silberchlorid AgCl aufweist.

2. Vorrichtung nach Anspruch 1, wobei die Messmittel (2) vier Arbeits-Mikroelektroden (41, 42, 43, 44) umfassen.

3. Vorrichtung nach Anspruch 2, wobei der Metalldraht (20) der Arbeits-Mikroelektroden (41, 42, 43, 44) aus Platin, Gold, Silber bzw. aus Wolfram oder Palladium ist.

4. Vorrichtung nach einem der Ansprüche 2 oder 3, wobei die vier Arbeitselektroden (41, 42, 43, 44) einen Kreis bilden, wobei die Mitte des Kreises durch die Referenz-Mikroelektrode (56) belegt ist.

5. Vorrichtung nach Anspruch 4, wobei der Kreis einen Radius in der Größenordnung von 1 cm hat, wobei der Messbereich kleiner als $10^{-2}$ $mm^2$ ist.

6. Vorrichtung nach einem der Ansprüche 1 bis 5, wobei der gemittelte Außendurchmesser (15) des freien Endes (7) jeder Arbeits-Mikroelektrode (4) im Wesentlichen gleich 120 $\mu$m ist, wobei der gemittelte Innendurchmesser (16) im Wesentlichen gleich 50 $\mu$m ist.

7. Verfahren zum Messen der physikalisch-chemischen Eigenschaften der Haut, insbesondere ihres pH-Wertes und ihres Oxidationszustands, mit Hilfe einer Messvorrichtung (1) nach einem der Ansprüche 1 bis 6, wobei das Verfahren einen Schritt der Bestimmung der pH-Werte und des Oxidationszustands in Abhängigkeit der Ergebnisse umfasst, die durch gleichzeitige Schritte erhalten werden, wonach:

- die Potentialdifferenz ($\Delta$E) bei null Strom (I) zwischen einer Arbeits-Mikroelektrode (4) und der Referenz-Mikroelektrode (56) gemessen wird;
- gleichzeitig für die anderen Mikroelektroden zumindest ein zyklisches Voltammogram zwischen den Potentialen von -0,8 V bis 1,5 V aufgenommen wird, um die Kurve des Elektrolysestroms (I) zu erhalten, der den Kreislauf durchläuft, der von der jeweiligen Arbeits-Mikroelektrode (4) und der Referenz-Mikroelektrode (56) gebildet wird, als Funktion der Potentialdifferenz ($\Delta$E), die zwischen der jeweiligen Arbeits-Mikroelektrode (4) und der Referenz-Mikroelektrode (56) angelegt wird.

8. Verfahren nach Anspruch 7, wobei vier Arbeits-Mikroelektroden (41, 42, 43, 44) mit dem Multipotentiostat (9) verbunden werden und gleichzeitig ein zyklisches Voltammogram für drei Mikroelektroden aufgenommen wird.

9. Verfahren nach einem der Ansprüche 7 oder 8, wobei der pH-Wert ausgehend von der Potentialdifferenz bei null Strom, gemessen zwischen einer Arbeits-Mikroelektrode (44) aus Wolfram oder Palladium und der Referenz-Mikroelektrode (56), die aus Silber besteht, bestimmt wird.

10. Verfahren nach einem der Ansprüche 7 bis 9, wobei die Gegenwart nachgewiesen wird:

- von Ascorbinsäure und von Harnsäure durch die Messung eines Oxidationsstroms, der an einer Mikroelektrode aus Platin (43) aufgezeichnet wird;
- von Glutathion durch die Messung eines Oxidationsstroms, der an einer Mikroelektrode aus Gold (42) aufgezeichnet wird; und
- von Chloridionen durch die Messung eines Oxidationsstroms, der an einer Mikroelektrode aus Silber (41) aufgezeichnet wird.

11. Verfahren nach Anspruch 10, wobei die Gegenwart quantiFiziert wird:

- von Ascorbinsäure durch die Messung des Oxidationsstroms, der an einer Mikroelektrode aus Platin (43) bei einem Potential von 0,6 V/E$_{ref}$ auf einem Voltammogram aufgezeichnet wird, das zwischen -0,4 V/E$_{ref}$ und 1,2 V/E$_{ref}$ aufgenommen wurde;
- von Harnsäure durch die Messung des Oxidationsstroms, der an der Mikroelektrode aus Platin (43) bei dem Potential von 0,9 V/E$_{ref}$ aufgenommen wurde, nach Abzug des Oxidationsstroms bei dem Potential von 0,6 V/E$_{ref}$, welcher der Ascorbinsäure entspricht;
- von Glutathion durch die Messung des Oxidationsstroms, der an einer Mikroelektrode aus Gold (42) bei dem Potential von 1,2 V/E$_{ref}$ auf einem Voltammogram aufgezeichnet wird, das zwischen -0,2 V/E$_{ref}$ und 1,5 V/E$_{ref}$ aufgenommen wurde; und
- von Chloridionen durch die Messung des Oxidationsstroms, der an einer Mikroelektrode aus Silber (41) bei dem Potential von 0,2 V/E$_{ref}$ auf einem Voltammogram aufgezeichnet wird, das zwischen -0,8 V/E$_{ref}$ und 0,6 V/E$_{ref}$ aufgenommen wurde.

12. Verfahren nach Anspruch 11, wobei zwischen jeder Messung die freien Enden (7) der Mikroelektroden (41, 42, 43, 44, 56) folgendermaßen vorbereitet werden:

- bei der Mikroelektrode (43) aus Platin werden Potentialzyklen mit einer Abtastrate von 50 mV/s zwischen -0,6 V/E$_{ref}$ und 1,0 V/E$_{ref}$ in einer phosphatgepufferten Lösung mit 0,1 mol/l bei pH=7,0 durchgeführt;
- bei der Mikroelektrode (42) aus Gold werden Potentialzyklen mit einer Abtastrate von 50 mV/s zwischen -0,5 V/E$_{ref}$ und 1,4 V/E$_{ref}$ in einer phosphatgepufferten Lösung mit 0,1 mol/l bei pH=7,0 durchgeführt;
- bei der Mikroelektrode (41) aus Silber werden Potentialzyklen mit einer Abtastrate von 50 mV/s zwischen -1,0 V/E$_{ref}$ und 0,4 V/E$_{ref}$ in einer Kaliumnitratlösung KNO$_3$ / HNO$_3$ mit 0,1 mol/l bei pH=3,5 durchgeführt;
- die Mikroelektrode (44) aus Wolfram oder Palladium wird in einer phosphatgepufferten Lösung mit 0,1 mol/l bei pH=7,0 über einen Zeitraum von zwei Minuten auf das Potential von 1,0 V/E$_{ref}$ gebracht;
- die Referenz-Mikroelektrode (56) wird in einer Kaliumchloridlösung NaCl mit 3 mmol/l bei pH=4 über einen Zeitraum von fünf Minuten auf eine Stromstärke von 30 nA gebracht, um einen Niederschlag von Silberchlorid AgCl zu bilden.

13. Verfahren nach einem der Ansprüche 7 bis 12, wobei die Messzeit kleiner als fünf Minuten, vorzugsweise kleiner als eine Minute ist.

FIG.1

FIG.2

FIG.3

FIG.4

a.

FIG.5a

b.

FIG.5b

c.

FIG.5c

FIG.6

FIG.8

a.

FIG.7a

b.

FIG.7b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• FR 2845264 A

**Littérature non-brevet citée dans la description**

• **RUFFIEN-CISZAK et al.** Exploration of the global antioxidant capacity of the stratum corneum by cyclic voltammetry. *JOURNAL OF PHARMACEUTICAL AND BIOMEDICAL ANALYSIS,* Août 2005